# EUROPÄISCHE PATENTSCHRIFT

(11) **EP 1 372 609 B1**
(45) Veröffentlichungstag und Bekanntmachung des Hinweises auf die Patenterteilung: **20.09.2006**
(21) Anmeldenummer: 02702281.3
(22) Anmeldetag: 18.01.2002
(51) Int. Cl.: A61K 9/14, A61K 35/10

(54) **TORFPRODUKTZUSAMMENSETZUNG, VERFAHREN ZU DEREN HERSTELLUNG UND VERWENDUNG**
PEAT PRODUCT COMPOSITION, METHOD FOR THE PRODUCTION AND USE THEREOF
COMPOSITION DE PRODUIT DE TOURBE, SON PROCEDE DE PRODUCTION ET SON UTILISATION

(30) Priorität: 19.01.2001 DE 10102229; 07.06.2001 DE 10127715
(43) Veröffentlichungstag der Anmeldung: 02.01.2004
(73) Patentinhaber: Humaderm Gmbh, 26603 Aurich (DE)
(72) Erfinder: FISSER, Arthur, 26605 Aurich (DE)
(74) Vertreter: Heiland, Karsten
(86) Internationale Anmeldenummer: PCT/EP2002/000477
(87) Internationale Veröffentlichungsnummer: WO 2002/056865

(56) Entgegenhaltungen:
- WO-A-92/16216
- DE-C- 4 316 347
- US-A- 4 698 225
- BILJETINA R: "Large-Scale Peat Feed Preparation" REPRINTS OF PAPERS-AMERICAN CHEMICAL SOCIETY, DIVISION OF FUEL CHEMISTRY, Bd. 29, Nr. 6, 1984, Seiten 150-159, XP008014014 Menlo Park, Ca

## Beschreibung

Die Erfindung betrifft eine Tablette aus verpresstem Torfpulver, ein Verfahren zum Herstellen der Tablette sowie deren Verwendung.

Moor bildet sich, wenn absterbende Pflanzen, wie Bäume, Sträucher, Blumen oder Gräser, besonders langsam unter Sauerstoffmangel zersetzt werden. Dieser Vorgang findet in Sümpfen oder Randgebieten von Gewässern bei niedrigen Temperaturen statt.

Unter Torf versteht man im biologischen Sinn die Humus-Form der Moore als Bodenauflage aus wenig zersetzten, konservierten Pflanzenresten. Frisch gestochener Torf enthält bis zu 90% Wasser und weist eine durchschnittliche Dichte von 1 auf. Er wird in der Regel auf einen Wassergehalt von 25 - 30% getrocknet, um im Gartenbau als Kultursubstrat, Düngemittel und zur Bodenstrukturverbesserung, als Brennstoff, Wärmedämmstoff, Streumaterial und Verpackungsmaterial verwendet zu werden.

Moor zählt, wie beispielsweise auch Fango, zur Gruppe der Peloide. Klassische Peloidbehandlungen begründen ihre Wirkung vor allem in der thermophysikalischen Eigenschaft der eingesetzten Medien, sodass insbesondere die äußerliche Anwendung von Moor/Torf beim menschlichen Körper im Vordergrund steht.

Im Bereich der Tierzucht wird Torfpaste zur Prophylaxe und Behandlung von Durchfallerkrankungen für Ferkel verwendet, siehe T. Lenk, A. Bendar, Mh. Vet.-Med. 44 (1989): 563-565, und A. Bendar et al., Mh. Vet.-Med. 45 (1990): 239-243. Aus dem Stand der Technik ist auch die Bedeutung von Torfmoor bei der Haltbarmachung von Lebensmitteln bekannt, siehe Carbohydrate Polymers 36 (1998): 335-347.

Eine Untersuchung von Humussubstanzen bezüglich ihres toxikologischen Verhaltens als Therapeutika für Tiere ist aus einem Bericht der Fakultät für Tiermedizin der Universität Utrecht, Niederlande aus dem Jahr 1999 bekannt.
Für die meisten therapeutischen Anwendungen beim Menschen wird Moor in unterschiedlichen Konzentrationen mit Wasser vermengt und erwärmt, insbesondere für äußerliche Anwendungen.

Von der Firma Heilmoorbad Neydharting GmbH, A-4654, Bad Wimsbach-Neydharting, Österreich sind beispielsweise Hautcremes, Shampoos, Zahnpasta und dergleichen bekannt. Ferner wird von der Firma Heilmoorbad Neydharting GmbH eine Moor-Trinkkur angeboten, die mit angewärmtem Wasser eingenommen werden soll und zur Magen- und Darmpflege, zur Aktivierung von Leber und Galle sowie zur allgemeinen Kräftigung eingesetzt werden kann.

Nachteilig bei einer solchen Trinkkur ist jedoch, dass diese in einer flüssigen und sehr verdünnten Form eingenommen werden muss, wodurch die Wirksamkeit einer solchen Trinkkur erheblich eingeschränkt wird. Eine direkte Einnahme der Trinkkur bzw. ein konzentriertes Auflösen derselben in Wasser erscheint nicht möglich.

Ferner muss die Trinkkur mehrmals täglich eine bestimmte Zeit, etwa eine halbe Stunde vor den Mahlzeiten eingenommen werden, was für den Einnehmenden mit einer gewissen Disziplin und einer Beeinträchtigung seines gewohnten Lebensrhythmusses verbunden ist.

Immunmodulierende Substanzen, d.h. solche Substanzen, die das Immunsystem von Mensch und Tier beeinflussen können, sind aus dem Stand der Technik bekannt. Eine Immunmodulation kann beispielsweise durch Antibiotika induziert werden, die jedoch in der Regel teuer und teilweise schädlich (resistenzinduzierend) sind. Eine Immunmodulation kann ebenfalls durch Probiotika (definierte lebende/abgetötete Bakterien der normalen Microflora bzw. durch deren Bestandteile, u.a. Lipoteichonsäure, MDP, bakterielle Zellwandkomplexe) über Schleimhaut assoziiertes lymphatisches Gewebe (MALT: Mucosa-associated lymphoid tissue) induziert werden. Bei der Verwendung von Probiotika zur Immunmodulation ist jedoch nicht immer eine Nebenwirkungsfreiheit oder hohe Effektivität zu beobachten.

Ein Torfprodukt ist aus der US-A-4 698 225 bekannt. Es handelt sich um Pelletts, die hergestellt sind aus gemahlenem und getrocknetem Torf in Verbindung mit einem Bindemittel. Die Torfbestandteile weisen eine Größe von etwa 760 bis 2540 µm sowie eine Feuchte von 5 bis 50% auf.

Aufgabe der vorliegenden Erfindung ist es, eine Zusammensetzung auf der Basis von Moor bereitzustellen, welche die Nachteile des Standes der Technik überwindet, insbesondere in einer hochkonzentrierten und somit hochwirksamen Form verabreicht und zusammen mit Mahlzeiten eingenommen werden kann. Ferner ist es Aufgabe der vorliegenden Erfindung, eine Zusammensetzung bereitzustellen, die kostengünstig herstellbar und nebenwirkungsfrei ist.

Erfindungsgemäß wird die Aufgabe durch die in den unabhängigen Ansprüchen genannten Merkmale gelöst. Das verwendete Torfpulver ist ein hochkonzentriertes Torfprodukt mit einem Feuchtegehalt von höchstens 10%.

Dabei ist vorgesehen, dass das Torfpulver eine Teilchengröße von etwa 10 µm bis etwa 100 µm aufweist.

Auch kann vorgesehen sein, dass Zusatzstoffe, wie Mineralien, Vitamine und/oder dergleichen, enthalten sind.

Eine das Verfahren betreffende Aufgabe wird gelöst durch ein Verfahren zum Herstellen von Tabletten, umfassend die folgenden Schritte:
- Pressen eines möglichst reinen Schwarztorfs,
- Trocknen des Torfs auf einen Feuchtegehalt von höchstens etwa 10%,
- Aufbrechen des Torfs und Befreien von äußeren Anhaftungen, Faseranteilen und dergleichen in einem ersten Siebprozess, um einen sauberen Kern zu erhalten,
- Zermahlen des sauberen Kerns des Torfs zu einem Pulver mit einer Teilchengröße von hauptsächlich etwa 10 µm bis etwa 100 µm,
- Abtrennen unerwünschter Bestandteile aus dem Pulver in einem zweiten Siebprozess,
- Verpressen des Pulvers ohne Zugabe von Bindemitteln zu Tabletten.

Dabei kann erfindungsgemäß vorgesehen sein, dass das Trocknen durch Zufuhr von Luft über einen Zeitraum von etwa 3 Jahren erfolgt. Insbesondere wird der gepresste Schwarztorf etwa 2-3 Monate in der Sonne und danach über etwa 3 Jahre durch Zufuhr freier Luft und nur gegen Regen abgedeckt getrocknet.

Ferner wird erfindungsgemäß vorgeschlagen, dass das Zermahlen in einer Hammermühle durchgeführt wird.

Das Abtrennen der unerwünschten Bestandteile kann unterstützt werden durch vorheriges Abblasen des Pulvers unter Ionisierung und Agglomeration der unerwünschten Bestandteile.

Die erfindungsgemäße Zusammensetzung, insbesondere hergestellt nach einem erfindungsgemäßen Verfahren, kann zur Prophylaxe und/oder Behandlung von Stoffwechselerkrankungen, Stoffwechselstörungen und/oder Erkrankungen oder Symptomen, die mit Stoffwechselstörungen zusammenhängen, insbesondere von Hauterkrankungen, wie chronischer Akne, Psoriasis, Neurodermitis und dergleichen, Rheuma und Magen-Darmerkrankungen, wie Morbus Crohn verwendet werden.

Des Weiteren kann die Zusammensetzung zur Immunmodulation bei Mensch und Tier verwendet werden.

Bevorzugt ist eine Verwendung der erfindungsgemäßen Zusammensetzung zur Infektprophylaxe, insbesondere autoimmunologischen, allergischen und rheumatologischen Diathese.

Eine erfindungsgemäße Verwendung ist femer dadurch gekennzeichnet, dass die Zusammensetzung als Lebensmittel- und/oder Futtermittelergänzungsstoff verabreicht wird.

Eine weitere erfindungsgemäße Verwendung ist dadurch gekennzeichnet, dass die Zusammensetzung als Arzneimittel im Human- und/oder Veterinärbereich verabreicht wird.

Hierzu zählt auch eine Verabreichung als Medizinprodukt nach dem deutschen Medizinproduktgesetz (MPG).

Eine weitere erfindungsgemäße Verwendung ist dadurch gekennzeichnet, dass die Zusammensetzung in einer in einem Trägermaterial dispergierten Form verabreicht wird.

Ferner kann vorgesehen sein, dass die Zusammensetzung in einer Dosis von 0,5 bis 5 g pro Tag verabreicht wird. Die Dosis gilt gleichermaßen für Menschen und Tiere, z.B. Kälber, Schweine, Hunde, Katzen.

Bei einer erfindungsgemäßen Verwendung kann die Tagesdosis der Zusammensetzung etwa 1 g betragen.

Der Erfindung liegt somit die überraschende Erkenntnis zugrunde, dass durch eine intensive Trocknung eines Moores/Torfes auf einen Feuchtegehalt von höchsten etwa 10% zum einen eine konzentrierte Aufnahme durch Mensch oder Tier möglich ist und zum anderen die Wirksamkeit deutlich erhöht werden kann. Insbesondere kann die erfindungsgemäße Zusammensetzung völlig unproblematisch mit Mahlzeiten oder (bei Tieren) zusammen mit dem Futter eingenommen werden, in dem diese Mahlzeit bzw. das Futter als Trägermaterial dient. Auf Grund der getrockneten Pulverform der erfindungsgemäßen Zusammensetzung ist es aber auch möglich, diese in Tabletten zu pressen, zu Dragees oder Pillen zu verarbeiten oder in Kapseln abzufüllen, was eine von Trägermaterial unabhängige Einnahme ermöglicht.

Die bevorzugte Tagesdosis von 0,5 bis 5 g, besonders bevorzugt 1 g, stellt dabei keine Probleme hinsichtlich der Einarbeitung in ein Trägermaterial oder der Verarbeitung zu einer Tablette, einem Dragee, einer Pille oder der Abfüllung in eine Kapsel dar. Somit ist eine einfache Einnahme der erfindungsgemäßen Zusammensetzung, sowohl bei Verwendung als Lebensmittel- oder Futtermittelergänzungsstoff als auch als Arzneimittel im Human- oder Veterinärbereich möglich.

Die erfindungsgemäße Zusammensetzung kann mit dem erfindungsgemäßen Verfahren in besonders wirkungsvoller Weise hergestellt werden. Selbstverständlich ist die Erfindung nicht auf ausschließlich mit diesem Verfahren hergestellte Produkte beschränkt, da auch eine auf andere Weise hergestellte Zusammensetzung, die ein hochkonzentriertes Torfprodukt mit einem Feuchtegehalt von höchstens etwa 10% umfasst, dieselben vorteilhaften Eigenschaften zeigen würde.

Die erfindungsgemäße Zusammensetzung wurde mit überraschendem Erfolg bei Stoffwechselerkrankungen und Stoffwechselstörungen eingesetzt, wobei sowohl eine innere Entschlackung und Entgiftung als auch eine Regulierung des pH-Wertes eine Rolle spielen dürfte, obgleich wir in dieser Hinsicht an keinerlei Theorie gebunden sein möchten. Positive Auswirkungen sind auch bei allen Erkrankungen oder Symptomen zu beobachten, die direkt oder indirekt, mit Stoffwechselstörungen zusammenhängen, beispielsweise bei einer großen Anzahl von Hauterkrankungen, angefangen bei chronischer Akne über Psoriasis zu Neurodermitis, aber auch bei Rheuma oder Magen-Darmerkrankungen, wie Morbus Crohn oder dergleichen.

Der Erfindung liegt weiterhin die überraschende Erkenntnis zugrunde, dass die erfindungsgemäße Zusammensetzung äußerst effektiv zur Immunmodulation bei Mensch und Tier eingesetzt werden kann, so dass insbesondere Antibiotikatherapien vermieden werden können. Auf Grund der geringen Kosten des Torfs ist zudem eine kostengünstige Therapie zur Immunmodulation möglich.

Die erfindungsgemäße Verwendung der Zusammensetzung zur Immunmodulation erfüllt sowohl prophylaktische (abwehrsteigernde Maßnahmen) als auch therapeutische Funktionen, unter anderem bei Erkrankungen infektiologischer und allergischer Genese. Eine Therapie auf der Basis der erfindungsgemäßen Zusammensetzung kann auch bei tumordestruktiven (aber antibiotischen) Therapien Symptome wie Diarrhöe, Bauchbeschwerden und Immunsuppression, die auf einer nachhaltigen Störung der Integrität/Zusammensetzung der mucosalen Mikroflora basieren, minimieren.

Weitere Merkmale und Vorteile der vorliegenden Erfindung ergeben sich aus der nachfolgenden Beschreibung, in der ein erfindungsgemäßes Verfahren und Untersuchungsergebnisse der Verwendung der erfindungsgemäßen Zusammensetzung zur Immunmodulation dargelegt werden.

Zur Herstellung einer erfindungsgemäßen Zusammensetzung bzw. eines entsprechenden Produkts wird Schwarztorf gestochen und gepresst. Die dabei entstehenden Torfstücke weisen eine etwa zylindrische äußere Form von 30 cm Länge und 10 cm Durchmesser auf und werden auf offener Fläche (Erdboden, Gras, Wiesen usw) ausgebreitet und etwa 2 - 3 Monate in der Sonne gelagert. Das Stechen erfolgt im Frühling oder zu Beginn des Sommers, damit für diese Phase der Trocknung genügend Sonneneinstrahlung zur Vefügung steht. Auch kann die UV-Strahlung von Bedeutung sein. Eine Abdeckung der Torfstücke ist nicht vorgesehen.
Anschließend werden die auf eine Restfeuchte von etwa 30% getrockneten Torfstücke in Hallen oder Scheunen gelagert. Wichtig ist eine gute Belüftung, etwa als Zugluft durch einander gegenüberliegende Gebäudeöffnungen, sowie eine Abdeckung gegen Regen.
Bevorzugt ist eine Lagerung in offenen Gitterboxen auf bekannten Euro-Paletten, die jeweils mit ausreichenden Abständen angeordnet sind. Diese zweite Phase der Trocknung erfolgt über 2 - 4 Jahre bis zu einer Restfeuchte im Torf von 5 - 10%. Während der Trocknung erfolgt keine künstliche Erwärmung der Torfstücke. Maßgeblich ist viel mehr die genannte gute Belüftung.

Die durch Trocknung geschrumpften Torfstücke weisen außen an ihrer Oberfläche Anhaftungen auf. Es handelt sich dabei im Wesentlichen um Gräser, Blätter, kleine Äste, die während der ersten Trocknungsphase an den Torfstücken haften geblieben sind. Daneben enthalten die Torfstücke feine und grobe Faseranteile.

In einem ersten Schritt werden die getrockneten Torfstücke insbesondere in einem Walzen-Shredder vorgebrochen auf eine Größe von 0,5 - 2 cm. Dabei fallen die Anhaftungen und gröbere Faseranteile ab und werden ausgesiebt. Das so erhaltene Zwischenprodukt wird als sauberer Kern (des Torfs) bezeichnet.

Die kleinen Torfstücke (sauberer Kern) werde in einer Mühle (Hammermühle) fein gemahlen. Bevorzugt wird eine Einstellung mit einer Maximalgröße von 2 mm Durchmesser. Die im Torf vorhandenen feinsten Fasern sind kleiner als die genannte maximale Größe, müssen aber gleichwohl abgetrennt werden. Möglichst ist dies durch lonisierung und Agglomeration der Fasern. Die beim Mahlen auftretenden Reibungskräfte bewirken eine lonisierung der Fasern. Diese ballen sich zu größeren Klumpen zusammen und können über einen nachfolgenden Siebprozess abgetrennt werden. Vorzugsweise wird ein Rüttel- und Taumelsieb verwendet, mit einer Siebgröße von etwa 1 mm.

Dass das Sieb passierende Torfpulver enthält hauptsächlich Komgrößen von 10 µm bis 100 µm, zum Teil auch geringfügig darüber und darunter. Größere Torfstücke (mehr als 1 mm, weniger als 2 mm) fallen nur wenige an. Diese können gegebenenfalls von den agglomerierten Fasern getrennt und nochmals dem Mahlprozess in der Hammermühle zugeführt werden.

Das erhaltene Torfpulver ist hinsichtlich seiner Restfeuchte von 5 - 10% und der Korngröße von hauptsächlich 10 µm - 100 µm besonders charakteristisch und als Medizinprodukt geeignet. Das Torfpulver wird bevorzugt zu Tabletten verarbeitet und zwar durch Verpressen und ohne Zugabe irgendwelcher Bindemittel oder anderer Stoffe. Die Tabletten weisen jeweils ein Gewicht von etwa 500 mg auf, bei einer Dicke von 3 - 6 mm, einem Durchmesser von 10 - 15 mm und einer Härte von 40 - 70 N. Gepresst werden die Tabletten mit einer Kraft von 10 - 30 kN, gegebenenfalls auch in 2 oder mehreren Pressschritten.

Die so erhaltenen Tabletten stellen ein hochreines Produkt dar und können Mensch und Tier während oder außerhalb der üblichen Nahrungsaufnahme verabreicht werden.

Die erfindungsgemäße Zusammensetzung wurde in vitro und in vivo auf proliferationsinduzierende und immunmodulierende Wirkungen überprüft. Dabei wurden Proliferations- und Aktivierungsstudien mit Immunzellen, Fibroblasten, Knochenmark- und Tumorzellen durchgeführt.

Die entsprechenden Zellen/Ziellinien für die Proliferationsstudien wurden gemäß den aus dem Stand der Technik bekannten Methoden gewonnen. Die Zellproliferation wurde mit Hilfe des TITERTEK Multiscan, Flow Laboratories, Helsinki, Finnland, durch Auszählen im Hämocytometer bzw. durch Zellzahlbestimmung im COULTER Counter, Beckmann-Coulter Co., Krefeld, Deutschland, bestimmt. Als Kontrollsubstanzen wurden dabei relevante Cytokine bzw. Wachstumsfaktoren (u.a. II-1, II-2, TNF-Alpha, CSFs) sowie Standardaktivatoren (u.a. PMA-lonomycin und Endotoxin/LPS) verwendet, um die Aktivität der erfindungsgemäßen Zusammensetzung mit etablierten Standards vergleichen zu können. Bei diesen Versuchen zeigte sich, dass die Aktivität der Zusammensetzung im Vergleich zu etablierten Standards erhöht war.

Die Zusammensetzung weist auch einen Einfluss auf die Cytokinsynthese auf. Zu diesem Zweck wurden definierte Immunzellen (Lymphozyten, Monozyten) in vitro mit der Zusammensetzung in diversen Konzentrationen inkubiert. Die Cytokinmessungen erfolgten mittels ELISA aus den Zellkulturüberständen bzw. mittels Durchflusscytometem (FACS, flourescence-activated cell sorter, Becton Dickinson, Heidelberg, Deutschland) als freigesetzte oder intrazelluläre Cytokine. Für die Zusammensetzung wurde auch eine immunaktivierende Wirkung im lymphatischen System durch qualitative Analyse definierter Aktivierungsmarker mittels monoklonaler Antikörper und Durchflusscytometrie (FACS) gefunden. Menschliche Lymphozyten konnten dabei aus der Separation von peripherem Blut freiwilliger Probanden mittels Standardmethodik gewonnen werden.

Mit der erfindungsgemäßen Zusammensetzung sind Monozyten bzw. Granulozyten aktivierbar, was durch Chemilumineszenzexperimente mit der erfindungsgemäßen Zusammensetzung gezeigt werden konnte. Zu diesem Zweck wurden Zelllinien bzw. die entsprechenden Zellen aus dem peripheren Blut isoliert und anschließend (a) direkt mit der Zusammensetzung inkubiert und stimuliert, (b) mit der Zusammensetzung sensibilisiert ("geprimt") und anschließend mit standardisierten Aktivatoren aktiviert.

Die in vivo-Aktivität der Zusammensetzung wurde im BALB/c-Mausmodel überprüft. Sowohl im immunkompetenten als auch im immunsupprimierten Organismus konnte ein Einfluss der Zusammensetzung festgestellt werden, auch eine Tumorenempfänglichkeit kann vorliegen. Die Zusammensetzung wurde für diese Untersuchungen den BAL/c-Mäusen oral/systemisch verabreicht, und am Ende der Versuchsperiode wurde den Versuchstieren peripheres Blut, peritoneale Makrophagen und Thymusdrüsen entnommen. Die Thymozyten wurden gemäß Standardmethoden ausgewertet. In diesem Zusammenhang konnte festgestellt werden, dass die Zusammensetzung einen positiven Einfluss auf die Proliferation, Ausreifung und Ausschwemmung lymphatischer Zellen aus dem Thymus sowie deren Präsenz und Aktivierungszustand im-peripheren Blut hat.

Weiterführende Untersuchungen mit der Zusammensetzung zeigten, dass ein immunsuppressiver Antibiotika-/Steroideffekt beeinflusst werden kann. Dabei konnte festgestellt werden, dass eine adjuvante Verabreichung der Zusammensetzung die thymusdestruktive Wirkung von Hydrocortison mildern kann. Bei der Verwendung der Zusammensetzung bei Mensch und Tier sind Anzeichen einer antitumoralen/antimetastatischen Wirkung zu erkennen.

Die erfindungsgemäße Zusammensetzung kann auch in Kombinationspräparaten eingesetzt werden, sofern die weiteren Inhaltsstoffe Wirkung und/oder Stabilität der erfindungsgemäßen Zusammensetzung nicht beeinträchtigen. So ist es beispielsweise möglich, Präparate einzusetzen, die die erfindungsgemäße Zusammensetzung - zur Unterstützung bwz. Erzielung ergänzender Wirkung - mit Enzymen und/oder Vitaminen kombinieren. Als Enzyme in solchen Kombinationspräperaten bieten sich beispielsweise die für systemische Enzymtherapie bekannten Proteinasen an, die in J. Leipner und R. Saller, "Systemic Enzyme Therapy in Oncology", Drugs 2000, 59 (4) 769-780, näher beschrieben sind. Weitere Kombinationspräperate, z.B. auch mit anderen Immunmodulatoren, sind möglich.

## Patentansprüche

1. Tablette zur Verwendung als Arzneimittel, Medizinprodukt, Lebensmittelergänzungsstoff und/oder Futtermittelergänzungsstoff, hergestellt aus verpresstem Torfpulver ohne Zugabe von Bindemitteln, wobei das Torfpulver eine Restfeuchte von höchstens 10% und eine Korngröße von hauptsächlich 10 µm bis 100 µm aufweist.

2. Tablette nach Anspruch 1, **dadurch gekennzeichnet, dass** außer dem Torfpulver keine weiteren Stoffe enthalten sind.

3. Tablette nach Anspruch 1, **gekennzeichnet durch** Zusatzstoffe wie Mineralien, Vitamine und dergleichen.

4. Verfahren zur Herstellung von Tabletten, insbesondere von Tabletten nach einem oder mehreren der Ansprüche 1 bis 3, umfassend die folgenden Schritte:
- Pressen von Schwarztorf,
- Trocknen des Torfs auf einen Feuchtegehalt von höchstens etwa 10%,
- Aufbrechen des Torfs und Befreien von äußeren Anhaftungen, Faseranteilen und dergleichen in einem ersten Siebprozess, um einen sauberen Kern zu erhalten,
- Zermahlen des sauberen Kerns des Torfs zu einem Pulver mit einer Teilchengröße von hauptsächlich etwa 10 µm bis etwa 100 µm,
- Abtrennen unerwünschter Bestandteile aus dem Pulver in einem zweiten Siebprozess,
- Verpressen des Pulvers ohne Zugabe von Bindemitteln zu Tabletten.

5. Verfahren nach Anspruch 4, **dadurch gekennzeichnet, dass** der Torf beim Zermahlen oder danach ionisiert wird und unerwünschte Bestandteile agglomerieren.

6. Verfahren nach Anspruch 4 oder 5, **dadurch gekennzeichnet, dass** das Trocknen durch Zufuhr von Luft über einen Zeitraum von etwa drei Jahren erfolgt.

7. Verfahren nach Anspruch 4 oder einem der weiteren Ansprüche, **dadurch gekennzeichnet, dass** der gepresste Schwarztorf etwa zwei bis drei Monate in der Sonne getrocknet und danach über etwa drei Jahre durch Zufuhr freier Luft und nur gegen Regen abgedeckt getrocknet wird.

8. Verfahren nach Anspruch 4 oder einem der weiteren Ansprüche, **dadurch gekennzeichnet, dass** das Zermahlen in einer Hammermühle durchgeführt wird.

9. Verfahren nach Anspruch 4 oder einem der weiteren Ansprüche, **dadurch gekennzeichnet, dass** das reine Pulver ohne Zugabe weiterer Stoffe zu Tabletten gepresst wird.

10. Tabletten nach einem der Ansprüche 1 bis 3, insbesondere hergestellt nach einem Verfahren der Ansprüche 4 bis 9, zur Verwendung bei den nachfolgenden Verfahren:
- zur Prophylaxe und/oder zur Behandlung von Stoffwechselerkrankungen, Stoffwechselstörungen und/oder Erkrankungen oder Symptomen, die mit Stoffwechselstörungen zusammenhängen, insbesondere von Hauterkrankungen, wie chronischer Akne, Psoriasis, Neurodermitis und dergleichen, Rheuma und Magen-Darmerkrankungen, wie Morbos Crohn,
- und/oder zur Infektprophylaxe, insbesondere autoimmunologischen, allergischen und rheumatologischen Diathese,
- und/oder zur Immunmodulation bei Mensch und/oder Tier.

## Claims

1. Tablet for use as pharmaceutical, medicinal product, food supplement and/or feed supplement, made of compressed peat powder without the addition of binders, the peat powder having a residual moisture of not more than 10% and a particle size of mainly 10 *µ*m to 100 *µ*m.

2. Tablet according to Claim 1, **characterized in that**, besides the peat powder, no further substances are present.

3. Tablet according to Claim 1, **characterized by** additives such as minerals, vitamins and the like.

4. Method of preparing tablets, in particular tablets according to one or more of Claims 1 to 3, comprising the following steps:
- pressing black peat,
- drying the peat to a residual moisture content of not more than approximately 10%,
- loosening the peat and freeing it from external adhering matter, fibre components and the like in a first screening process in order to obtain a pure core,
- grinding the pure peat core to a powder with a particle size of mainly approximately 10 *µ*m to approximately 100 *µ*m,
- removing undesired constituents from the powder in a second screening process,
- compressing the powder to tablets without addition of binders.

5. Method according to Claim 4, **characterized in that** upon grinding or thereafter the peat is ionized and undesired constituents agglomerate.

6. Method according to Claim 4 or 5, **characterized in that** the drying process is carried out by supplying air over a period of approximately three years.

7. Method according to Claim 4 or one of the further claims, **characterized in that** the compressed black peat is dried in the sun for approximately two to three months and thereafter for approximately three years by supplying atmospheric air and only providing a rain cover.

8. Method according to Claim 4 or one of the further claims, **characterized in that** the grinding procedure is carried out in a hammer mill.

9. Method according to Claim 4 or one of the further claims, **characterized in that** the pure powder is compressed to tablets without the addition of further substances.

10. Tablets according to one of Claims 1 to 3, in particular prepared by a method of Claims 4 to 9, for use in the following procedures:
- for the prophylaxis and/or treatment of metabolic diseases, metabolic disorders and/or diseases or symptoms which are associated with metabolic disorders, in particular skin diseases such as chronic acne, psoriasis, neurodermitis and the like, rheumatism and diseases of the gastrointestinal tract, such as Crohn's disease,
- and/or for the prophylaxis of infections, in particular autoimmunological, allergic and rheumatological diathesis,
- and/or for the immune modulation in humans and/or animals.

## Revendications

1. Comprimé pour utilisation en tant que médicament, produit médical, complément alimentaire et/ou complément pour aliment pour animaux, fabriqué à partir de poudre de tourbe pressée sans addition de liants, la poudre de tourbe ayant une humidité résiduelle de 10 % au maximum et une taille de grain allant principalement de 10 *µm* à 100 *µ*m.

2. Comprimé selon la revendication 1, **caractérisé en ce qu'**aucune autre substance n'est contenue en plus de la poudre de tourbe.

3. Comprimé selon la revendication 1, **caractérisé par** des additifs tels que des minéraux, des vitamines et similaires.

4. Procédé pour la fabrication de comprimés, en particulier de comprimés selon une ou plusieurs des revendications 1 à 3, comprenant les étapes suivantes :
- pressage de tourbe noire,
- séchage de la tourbe jusqu'à une teneur en humidité d'environ 10 % au maximum,
- concassage de la tourbe et séparation des parties adhérentes externes, des parties fibreuses et similaires, dans un premier processus de tamisage, pour obtenir un coeur propre,
- broyage du coeur propre de la tourbe en une poudre ayant une taille de particule allant principalement d'environ 10 *µ*m à environ 100 *µ*m,
- séparation des composants indésirables de la poudre dans un second processus de tamisage,
- pressage de la poudre sans addition de liants, en comprimés.

5. Procédé selon la revendication 4, **caractérisé en ce que** la tourbe est ionisée lors du broyage ou après le broyage et les composants indésirables s'agglomèrent.

6. Procédé selon la revendication 4 ou 5, **caractérisé en ce que** le séchage s'effectue par apport d'air pendant une durée d'environ trois ans.

7. Procédé selon la revendication 4 ou l'une quelconque des revendications suivantes, **caractérisé en ce que** la tourbe noire pressée est séchée au soleil pendant environ deux à trois mois et est ensuite séchée pendant trois ans environ par apport d'air libre et en étant seulement protégée contre la pluie.

8. Procédé selon la revendication 4 ou l'une quelconque des revendications suivantes, **caractérisé en ce que** le broyage est effectué dans un broyeur à marteaux.

9. Procédé selon la revendication 4 ou l'une quelconque des revendications suivantes, **caractérisé en ce que** la poudre pure est pressée en comprimés sans addition d'autres substances.

10. Comprimés selon l'une quelconque des revendications 1 à 3, en particulier fabriqués selon un procédé des revendications 4 à 9, pour utilisation dans les procédés suivants :
- pour la prophylaxie et/ou pour le traitement de maladies métaboliques, de troubles du métabolisme et/ou de maladies ou symptômes qui sont en rapport avec des troubles du métabolisme, en particulier de maladies de peau telles que l'acné chronique, le psoriasis, la névrodermite et similaires, les rhumatismes et les maladies gastrointestinales telles que la maladie de Crohn.
- et/ou pour la prophylaxie de l'infection, en particulier la diathèse auto-immune, allergique ou rhumatismale,
- et/ou pour l'immunomodulation chez l'homme et l'animal.
